(19) Europäisches Patentamt
European Patent Office
Office européen des brevets

(11) **EP 1 862 185 B1**

(12) **EUROPÄISCHE PATENTSCHRIFT**

(45) Veröffentlichungstag und Bekanntmachung des Hinweises auf die Patenterteilung:
**15.04.2009 Patentblatt 2009/16**

(51) Int Cl.:
*A61L 2/025* (2006.01)    *A61L 2/10* (2006.01)
*C02F 1/32* (2006.01)    *C02F 1/36* (2006.01)

(21) Anmeldenummer: **07007702.9**

(22) Anmeldetag: **16.04.2007**

(54) **Vorrichtung zur Aufbereitung von Flüssigkeiten mittels UV-Licht und Ultraschall**

Device for treating fluids using UV light and ultrasound

Dispositif destiné à la traitement de liquides par lumière UV et ultrason

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **31.05.2006 DE 202006008743 U**
**06.06.2006 DE 102006026569**

(43) Veröffentlichungstag der Anmeldung:
**05.12.2007 Patentblatt 2007/49**

(73) Patentinhaber: **HeliosAquaPlus Technology AG**
**28355 Bremen (DE)**

(72) Erfinder:
• **Riggers, Wolfgang**
**28355 Bremen (DE)**
• **Heyer, Uwe**
**27568 Bremerhaven (DE)**

(74) Vertreter: **Manasse, Uwe et al**
**Forrester & Boehmert**
**Pettenkoferstrasse 20-22**
**80336 München (DE)**

(56) Entgegenhaltungen:
**DE-U1-3202005 009 92**    **DE-U1-6202006 001 07**
**DE-U1-3202006 008 74**    **US-A1- 2003 194 692**
**US-A1- 2005 042 129**

• **[Online] 1. September 2004 (2004-09-01), , XP002448207 Gefunden im Internet: URL:http:// edocs.tu-berlin.de/diss/2004/ze nker_ marco.pdf> [gefunden am 2007-08-27] * das ganze Dokument ***

**Beschreibung**

Die vorliegende Erfindung betrifft Vorrichtungen zur Aufbereitung von Flüssigkeiten zwecks Sterilisation und Elimination von darin enthaltenen organischen Stoffen mittels UV-Licht.

Im Rahmen vieler Anwendungen ist eine hohe Reinheit von Flüssigkeiten eine absolute Notwendigkeit. Eine adäquate Flüssigkeitsaufbereitung ist in der Praxis jedoch aufgrund von fehlenden technischen Realisierungsmöglichkeiten nur allzu oft nicht möglich. Dabei meint adäquate Flüssigkeitsaufbereitung eine Eliminierung problematischer Mikroorganismen, wie zum Beispiel Keime, Viren, Pilze etc., und Pyrogene, wie zum Beispiel Endotoxine, Exotoxine etc. Zu erwähnen sind zudem Schadstoffe, wie Pestizide etc. Dies gilt insbesondere bei Anwendungen, die wässrige Flüssigkeiten benötigen, die während der Anwendung zuvor unzureichend aufbereitet bezogen werden.

Derzeit ist die Herstellung von sogenannten "reinen" Flüssigkeiten oft nur unter Verwendung von Filtern, wie zum Beispiel in Kombination mit einem Aktivkohlefilter, möglich. Dieser Aufbereitungsschritt kann jedoch sehr kostenintensiv sein und bietet langfristig aufgrund der Gefahr der Bildung multiresistenter Keime keine befriedigende Lösung.

Es sind Geräte bekannt, die die Verwendung von Chlor und von anderen Chemikalien vermeiden, indem sie zur Wasserentkeimung die Bestrahlung mit UV-Licht bestimmter Wellenlängen oder Ultraschall vorsehen. Das, insbesondere kurzwellige, UV-Licht wird in Substanzen, wie zum Beispiel DNA, in der Flüssigkeit absorbiert. Die absorbierte UV-Lichtenergie reicht aus, um eine photochemische Umwandlung zu bewirken. Eine für die Teilung der DNA notwendige Informationsweitergabe unterbleibt. Bei der Überschreitung eines Informationsstörungsniveaus sterben Zellen, ohne sich zu vermehren. Somit werden durch UV-Licht lebende Mikroorganismen durch Zerstörung der DNA abgetötet oder inaktiviert, das heißt, daß dadurch eine sterilisierende Wirkung erzielt wird. Die Wellenlänge des UV-Lichtes liegt hierbei typischerweise in einem Bereich von 70 bis 400 nm, vorzugsweise bei 254 bzw. 180 bis 200 nm bei Vakuum-UVC-Lampen.

Der Wirkungsgrad der Vorrichtungen, die eine Sterilisation mittels UV-Licht durchführen, ist jedoch noch ungenügend.

DE 20 2005 009923 U1 offenbart eine Vorrichtung gemäß der Präambel von Anspruch 1.

Der Erfindung liegt somit die Aufgabe zugrunde, eine Vorrichtung zur Aufbereitung von Flüssigkeiten zwecks Sterilisation und Elimination von darin enthaltenen organischen Stoffen mittels UV-Licht mit einem höheren Wirkungsgrad zu schaffen.

Gemäß einem ersten Aspekt wird diese Aufgabe gelöst durch eine Vorrichtung gemäß Anspruch 1.

Bei der Vorrichtung gemäß dem ersten Aspekt kann vorgesehen sein, daß die Entgasungseinrichtung eine mechanische Entgasungseinrichtung umfaßt.

Weiterhin ist denkbar, daß die Entgasungseinrichtung einen mechanischen Luftabscheider umfaßt.

Gemäß einer besonderen Ausführungsform der Erfindung umfaßt die Entgasungseinrichtung eine Ultraschallquelle, einen Membrankontaktor, eine Vakuum-Entgasungseinrichtung oder eine Düse in dem Flüssigkeitszulauf.

Weiter ist vorgesehen daß das Gehäuse die Gestalt eines Zylinders aufweist und in Längsrichtung des Zylinders in zwei Kammern unterteilt ist, sich die UV-Lichtquelle in Längsrichtung des Zylinders durch beide Kammern erstreckt, ein erstes für UV-Licht durchlässiges Schutzrohr die UV-Lichtquelle durch beide Kammern hindurch umgibt, ein zweites Rohr mit einem größeren Innendurchmesser als der Außendurchmesser des ersten Schutzrohres das erste Schutzrohr unter Bildung eines Ringspalts durch beide Kammern hindurch umgibt, wobei an beiden Enden des zweiten Rohres jeweils mindestens eine Öffnung zur Herstellung einer Flüssigkeitsverbindung zwischen dem Ringspalt und der Außenseite des zweiten Rohres vorgesehen ist und der Flüssigkeitszulauf fern von der mindestens einen Öffnung an einem Ende des zweiten Rohres in einer der beiden Kammern vorgesehen ist und der Flüssigkeitszulauf fern von der mindestens einen Öffnung an dem anderen Ende des zweiten Rohres in der anderen Kammer vorgesehen ist. In dem Ringspalt, dem eigentlichen UV-Behandlungsbereich, wird die aufzubereitende Flüssigkeit einer besonders hohen Dosis von UV-Strahlung ausgesetzt. Da die Eindringtiefe bei trüben oder gefärbten Flüssigkeiten für UV-Licht sehr niedrig sein kann, kann durch die Breite des Ringspaltes für jede aufzubereitende Flüssigkeit die optimale Durchdringungstiefe für UV-Licht erreicht werden. Dies liefert eine höhere sterilisierende Wirkung.

Günstigerweise ist die mindestens eine Ultraschallquelle zur Emission von Ultraschall mit einer Frequenz > 18 kHz und/oder einer Leistung > 1 Watt/cm$^2$ gestaltet.

Vorteilhafterweise ist die mindestens eine Ultraschallquelle oder mindestens eine Ultraschallquelle so angeordnet, daß sie im Betrieb im wesentlichen senkrecht zur Längsrichtung des Zylinders Ultraschall vom Gehäuse in Richtung auf die UV-Lichtquelle einstrahlt.

Ebenfalls ist günstigerweise mindestens eine Ultraschallquelle so angeordnet, daß sie im Betrieb im wesentlichen parallel zur Längsrichtung des Zylinders Ultraschall im Bereich zwischen dem zweiten Rohr und dem Gehäuse einstrahlt.

Vorteilhafterweise ist jeder der beiden Kammern jeweils mindestens eine Ultraschallquelle zum Bestrahlen der aufzubereitenden Flüssigkeit mit Ultraschall zugeordnet.

Besonders bevorzugt befindet sich in der Kammer mit dem Flüssigkeitszulauf mindestens ein Kavitation erzeugendes Sieb oder Filter. Mit Kavitation ist eine Hohlraumbildung in Flüssigkeiten gemeint. Eine Hohlraumbildung setzt ein, wenn der örtliche statische Druck in einer Flüssigkeit unter einen kritischen Wert absinkt. Dieser kritische Wert ist in der Regel etwa gleich groß wie der Dampfdruck der Flüssigkeit. Kavitation kann als Strömungskavitation zum Beispiel durch Filter, Schwingungskavitation zum Beispiel durch Ultraschall und Siedekavitation zum Beispiel durch Erhitzung auftreten. Strömungskavitation tritt hauptsächlich in Querschnittsverengungen, Querschnittserweiterungen oder plötzlichen Umlenkungen der Strömungsrichtung auf. An Querschnittsverengungen sinkt der statische Druck aufgrund der Vergrößerung der Strömungsgeschwindigkeit und damit der Umwandlung statischen Drucks in dynamischen Druck (Strömungsenergie). Hinter Querschnittserweiterungen treten meist Wirbel auf, in deren Zentrum wegen der hohen Umfangsgeschwindigkeit der Flüssigkeit der statische auf den kritischen Wert absinken kann. Ein ähnlicher Effekt tritt hinter plötzlichen Umlenkungen auf. Man unterscheidet auch zwischen Dampf- und Gaskavitation, je nachdem, ob sich in den Kavitationsblasen Flüssigkeitsdampf oder ein Gas, zum Beispiel Luft, befindet, das in der Flüssigkeit gelöst war. Beispielsweise löst Wasser nur geringe Mengen an Luft. Deshalb wird bei Kavitation in Wasser im allgemeinen Dampfkavitation vorliegen.

Bei Filtern und Sieben tritt auch ein Düseneffekt, das heißt Kavitation wie bei Düsen und kleinen Öffnungen, auf. Die Struktur des Filters bzw. Siebs erhöht die Strömungsgeschwindigkeit an den Grenzflächen desselben, so daß Kavitation entsteht. Durch diese Kavitation entstehen in den aufzubereitenden Flüssigkeiten Kavitationsblasen aus in der Flüssigkeit gelösten Gasen und aus Wasserdampf, die bei Fehlen von gelösten Gasen in der Flüssigkeit entstehen. Die Wasserdampfblasen kollabieren und erzeugen dabei Energien, die wiederum Radikale, OH-Radikale, erzeugen. Diese Radikale sind in der Lage, organische Strukturen zu zerstören. Des weiteren verbinden sich OH-Radikale zu $H_2O_2$, das heißt Wasserstoffperoxid, das als starker Oxidator von organischen Stoffen bekannt ist. Der Nachweis ist in der Literatur von Alois Jungbauer, Universität Wien, beschrieben. Es wird destilliertem Wasser Kaliumjodid zugeführt. OH-Radikale führen zu einer chemischen Reaktion, so daß Jod entsteht, das leicht nachgewiesen werden kann und gleichzeitig als Indikator für den Wirkungsgrad des angewandten Verfahrens gilt.

Günstigerweise befindet sich das mindestens eine Sieb bzw. Filter in der Kammer im Bereich des Flüssigkeitszulaufs.

Insbesondere kann vorgesehen sein, daß die Entgasungseinrichtung eine mechanische Entgasungseinrichtung, insbesondere einen mechanischen Luftabscheider, umfaßt.

Vorteilhafterweise umfaßt die Entgasungseinrichtung eine Ultraschallquelle, einen Membrankontaktor, eine Vakuum-Entgasungseinrichtung oder eine Düse in dem Flüssigkeitszulauf.

Es ist vorgesehen, daß das Gehäuse die Gestalt eines Zylinders aufweist und in Längsrichtung des Zylinders in zwei Kammern unterteilt ist, sich die UV-Lichtquelle in Längsrichtung des Zylinders durch beide Kammern erstreckt, ein erstes für UV-Licht durchlässiges Schutzrohr die UV-Lichtquelle durch beide Kammern hindurch umgibt, ein zweites Rohr mit einem größeren Innendurchmesser als der Außendurchmesser des ersten Schutzrohres das erste Schutzrohr unter Bildung eines Ringspalts durch beide Kammern hindurch umgibt, wobei an beiden Enden des zweiten Rohres jeweils mindestens eine Öffnung zur Herstellung einer Flüssigkeitsverbindung zwischen dem Ringspalt und der Außenseite des zweiten Rohres vorgesehen ist und der Flüssigkeitszulauf fern von der mindestens einen Öffnung an einem Ende des zweiten Rohres in einer der beiden Kammern vorgesehen ist und der Flüssigkeitszulauf fern von der mindestens einen Öffnung an dem anderen Ende des zweiten Rohres in der anderen Kammer vorgesehen ist.

Vorteilhafterweise ist die mindestens eine Ultraschallquelle zur Emission von Ultraschall mit einer Frequenz > 18 kHz und/oder einer Leistung > 1 Watt/cm$^2$ gestaltet.

Günstigerweise ist die mindestens eine Ultraschallquelle oder mindestens eine Ultraschallquelle so angeordnet, daß sie im Betrieb im wesentlichen senkrecht zur Längsrichtung des Zylinders Ultraschall vom Gehäuse in Richtung auf die UV-Lichtquelle einstrahlt.

Zweckmäßigerweise ist mindestens eine Ultraschallquelle so angeordnet, daß sie im Betrieb im wesentlichen parallel zur Längsrichtung des Zylinders Ultraschall im Bereich zwischen dem zweiten Rohr und dem Gehäuse einstrahlt.

Außerdem kann vorgesehen sein, daß jeder der beiden Kammern jeweils mindestens eine Ultraschallquelle zum Bestrahlen der aufzubereitenden Flüssigkeit mit Ultraschall zugeordnet ist.

Schließlich kann vorteilhafterweise bei der Vorrichtung gemäß dem ersten oder zweiten Aspekt der Erfindung die Entgasungseinrichtung innerhalb des Gehäuses, vorzugsweise im Bereich des Flüssigkeitszulaufs, vorgesehen sein.

Der Vorrichtung gemäß dem ersten Aspekt liegt die überraschende Erkenntnis zugrunde, daß insbesondere durch die Entgasungseinrichtung der Wirkungsgrad der Vorrichtung erhöht wird. Durch die Entgasung entstehen mehr OH-Radikale durch anteilig höhere Dampfkonzentration, die sich zu Wasserstoffperoxid ($H_2O_2$) verbinden. Beim nachfolgenden kombinierten Einsatz von UV-Licht und Wasserstoffperoxid, wobei letzteres auch durch die mindestens eine Ultraschallquelle als Kavitationsquelle erzeugt wird, finden unter anderem folgende Reaktionen statt:

Photolyse des Wasserstoffperoxids unter Bildung von OH-Radikalen:

Wasserstoffperoxid besitzt ein kontinuierliches Absorptionsspektrum im UV-Wellenlängenbereich. Dabei nimmt die Absorption mit abnehmender Wellenlänge zu.

$$H_2O_2 + h\nu = 2\, OH^\bullet$$

Oxidation eines Schadstoffmoleküls M durch die OH-Radikale:

$$OH^\bullet + M = M_{ox}.$$

Zum Beispiel wird die aufzubereitende Flüssigkeit beim Einströmen in das Gehäuse mittels einer Ultraschallquelle entgast, zum Beispiel durch Druckunterschiede der durchströmenden Flüssigkeit. Andererseits können auch beispielsweise Düsen verwendet werden. Der dabei entstehende Druckabfall entgast die aufzubereitende Flüssigkeit, da ein Druckgefälle zum Atmosphärendruck erzeugt wird.

Durch die mindestens eine Ultraschallquelle in Kombination mit der Entgasungseinrichtung wird also besonders viel Wasserstoffperoxid erzeugt, das den oben geschilderten Reaktionen unterliegt.

Wenn in einer besonderen Ausführungsform die aufzubereitende Flüssigkeit in einem Ringspalt an einer UV-Lichtquelle vorbei geführt wird, so kann das UV-Licht besonders tief und effizient in die aufzubereitende Flüssigkeit eindringen und wirksam werden.

Wenn gemäß einer weiteren besonderen Ausführungsform ein Kavitation erzeugendes Sieb bzw. Filter vorgesehen ist, so wird durch die - zu der durch die mindestens eine Ultraschallquelle erzeugte Kavitation zusätzliche - Kavitation der Wirkungsgrad weiter erhöht.

Der Vorrichtung gemäß dem zweiten Aspekt liegt die überraschende Erkenntnis zugrunde, daß mittels eines Kavitation erzeugenden Siebs bzw. Filters grundsätzlich von der Verwendung einer Ultraschallquelle abgesehen werden könnte.

Weitere Merkmale und Vorteile der Erfindung ergeben sich anhand der beigefügten Ansprüche und der nachfolgenden Beschreibung, in der drei Ausführungsbeispiele anhand der schematischen Zeichnungen im einzelnen erläutert sind, in denen:

Figur 1    eine dimetrische Darstellung einer Vorrichtung zur Aufbereitung von Flüssigkeit zwecks Sterilisation und Elimination von darin enthaltenen organischen Stoffen mittels UV-Licht gemäß einer ersten besonderen Ausführungsform der Erfindung zeigt;

Figur 2    eine Längsschnittansicht der Vorrichtung von Figur 1 zeigt;

Figur 3    eine Längsschnittansicht durch eine Vorrichtung gemäß einer zweiten besonderen Ausführungsform der Erfindung zeigt; und

Figur 4    eine Längsschnittansicht durch eine Vorrichtung gemäß einer dritten besonderen Ausführungsform der vorliegenden Erfindung zeigt;

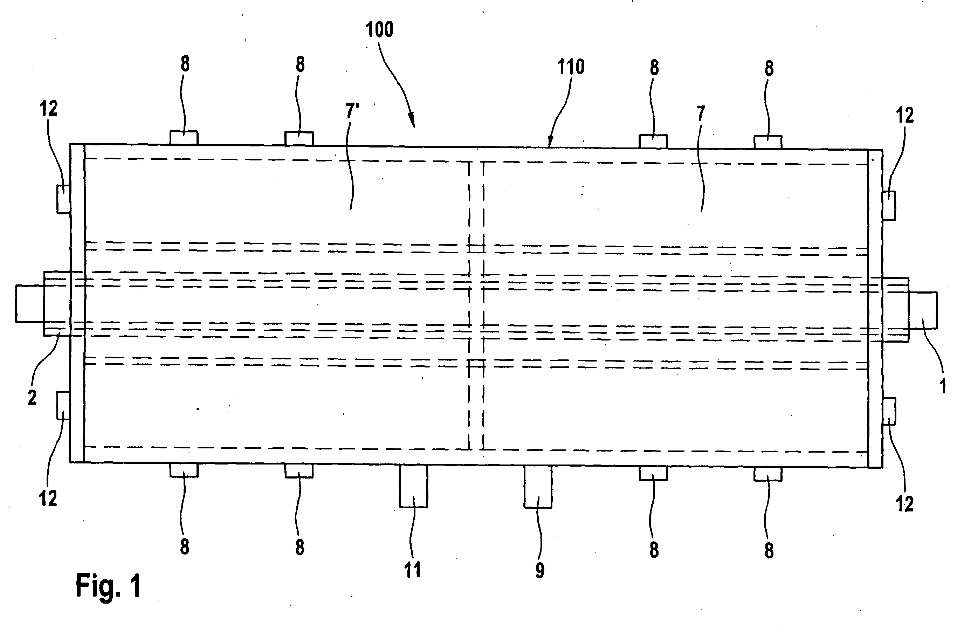

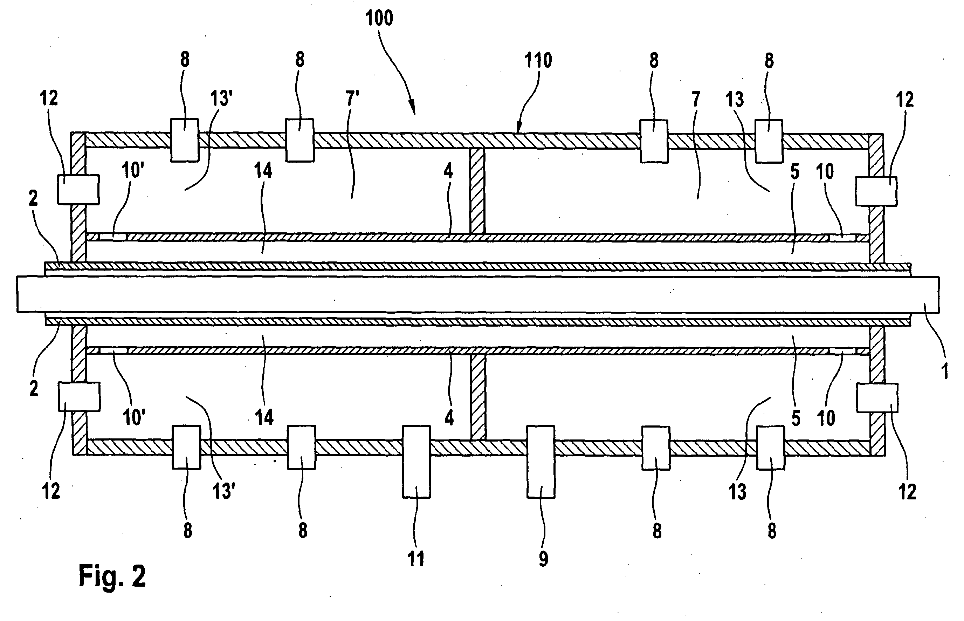

Die in Figur 1 gezeigte Vorrichtung 100 weist ein einteiliges zylindrisches Gehäuse 110 auf, das in Längsrichtung in zwei gleichgroße Kammern 7, 7' unterteilt ist. In der Kammer 7 ist ein Flüssigkeitszulauf 9 und in der Kammer 7' ist ein Flüssigkeitsablauf 11 vorgesehen. Der Flüssigkeitszulauf 9 und der Flüssigkeitsablauf 11 befinden sich jeweils in Längsrichtung des zylinderförmigen Gehäuses 110 in der Nähe der Mitte desselben. Wie sich unter Hinzuziehung der Figur 2 ergibt, erstreckt sich in der Mitte des Gehäuses 110 in dessen Längsrichtung eine UV-Lichtquelle 1 durch beide Kammern 7, 7' hindurch. Ein erstes für UV-Licht durchlässiges Schutzrohr 2 umgibt die UV-Lichtquelle 1 durch beide Kammern 7, 7' hindurch. Ein zweites Rohr 4 mit einem größeren Innendurchmesser als der Außendurchmesser des ersten Schutzrohres 2 umgibt das erste Schutzrohr unter Bildung eines Ringspalts 5 durch beide Kammern 7, 7' hindurch. An beiden Enden des zweiten Rohres 4 befindet sich jeweils eine Öffnung 10 bzw. 10' zur Herstellung einer Flüssigkeitsverbindung zwischen dem Ringspalt 5 und der Außenseite des zweiten Rohres 4. In dem Gehäuse 110 sind in beiden Kammern 7, 7' jeweils Ultraschallquellen 8, die von der Gehäusewand zumindest im wesentlichen senkrecht zur Längsrichtung des Zylinders nach innen Ultraschall abstrahlen, und Ultraschallquellen 12, die Ultraschall parallel zur Längsrichtung des zylinderförmigen Gehäuses zwischen dem zweiten Rohr 4 und dem Gehäuse 110 abstrahlen, vorgesehen. Demzufolge werden die Bereiche zwischen dem Gehäuse 110 und dem zweiten Rohr 4 als Ultraschallbehandlungsbereiche 13, 13' und der Bereich zwischen dem zweiten Rohr 4 und dem ersten Schutzrohr 2 als UV-Behandlungsbereich 14 bezeichnet. Die aufzubereitende Flüssigkeit fließt somit durch den Flüssigkeitszulauf 9 in den Ultraschallbehandlungsbereich 13, dann weiter durch die Öffnungen 10 in den UV-Behandlungsbereich 14, dann weiter durch die Öffnungen 10' in den Ultraschallbehandlungsbereich 13' und danach durch den Flüssigkeitsablauf 9 aus dem Gehäuse 110 heraus. In den Ultraschallbehandlungsbereichen 13, 13' kann die aufzubereitende Flüssigkeit mit Ultraschall effektiv beaufschlagt werden und in dem UV-Behandlungsbereich 14 kann sie effektiv mit UV-Licht bestrahlt werden. In den Ultraschallbehandlungsbereichen 13, 13' kommt es durch den Ultraschall zur Ultraschallabtötung als Hauptursache für den mechanischen Zellaufbruch. Neben der mechanischen Erosion der Zellwand induzieren die starken Druck-Temperatur-Wechsel Polymerisierungsreaktionen im Inneren der Zelle, die zum Abbau von globulären Proteinen führen. Diese werden in ihre Untereinheit aufgespalten, wobei zunächst deren Quartärstruktur zerstört wird. In diesem Zusammenhang sind neben der Abtrennung von niedrig molekularen Peptiden die Aufspaltung von zyklischen Aminosäuren beobachtet worden.

Durch mechanische Erosion der Zellwände wurde eine erhöhte UVC-Absorption von Zellen festgestellt, wodurch im UV-Behandlungsbereich ein erhöhter Wirkungsgrad erreicht wird.

Durch starke Druckunterschiede durch Ultraschall werden Schadstoffverbände deagglomeriert und homogen gehalten, wodurch im UV-Bereich die Strahlung wirkungsvoller Schadstoffe erreicht und eliminieren kann. Der von den Ultraschallquellen 8 erzeugte Ultraschall durchdringt das zweite Rohr 4 und dringt in den flüssigkeitsführenden UV-Behandlungsbereich 14 ein. Auf diese Weise kommt es auch im UV-Behandlungsbereich zur mechanischen Ultraschallabtötung von Schadstoffen, einer mechanischen Erosion der Zellwände und dadurch zu einer erhöhten UVC-Absorption von Zellen, wodurch im UV-Behandlungsbereich ein erhöhter Wirkungsgrad und starke Druckunterschiede durch Ultraschall erreicht werden, wodurch in diesem Bereich Schadstoffverbände deagglomeriert und homogen gehalten werden, wodurch im UV-Behandlungsbereich die Strahlung wirkungsvoller Schadstoffe erreicht und eliminieren kann.

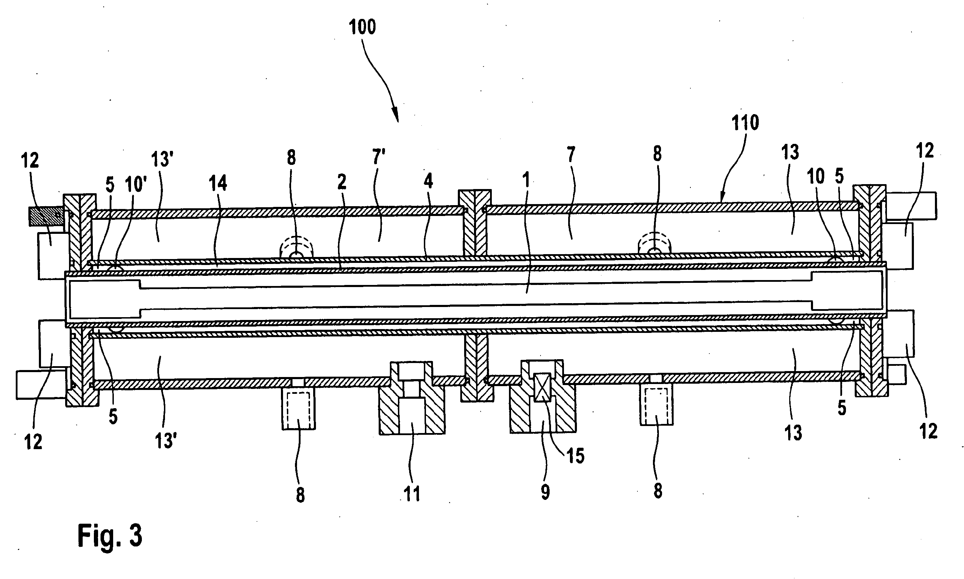

Die in Figur 3 gezeigte Vorrichtung unterscheidet sich von der in den Figuren 1 und 2 gezeigten Vorrichtungen im wesentlichen dadurch, daß im Flüssigkeitszulauf 9 eine Entgasungseinrichtung 15 vorgesehen ist. Die Entgasungseinrichtung dient zur Entgasung der in das Gehäuse 110 über den Flüssigkeitszulauf strömenden, aufzubereitenden Flüssigkeit.

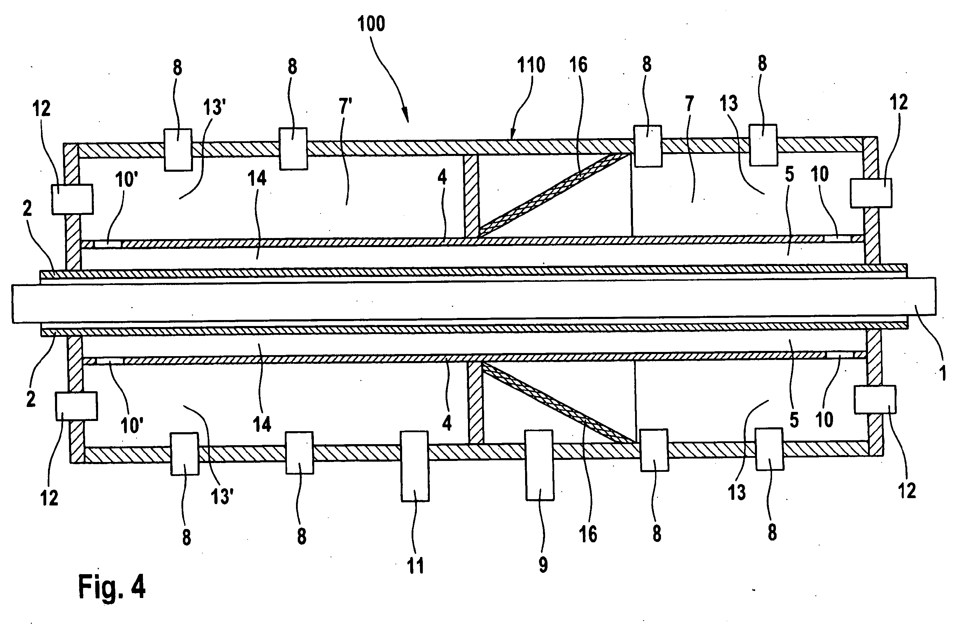

Schließlich unterscheidet sich die in der Figur 4 gezeigte Vorrichtung von der in den Figuren 1 und 2 gezeigten Vorrichtung im wesentlichen darin, daß in der Kammer 7 mit dem Flüssigkeitszulauf 9 ein Kavitation erzeugendes Sieb 16 vorgesehen ist, das aufzubereitende Flüssigkeit, die in das Gehäuse über den Flüssigkeitszulauf 9 einströmt, sozusagen als erstes passieren muß. Das Sieb 16 erstreckt sich vom Gehäuse 110 schräg nach innen in Richtung auf die UV-Lichtquelle 1.

Die vorliegende Erfindung liefert erstmalig Vorrichtungen, bei denen kein Wasserstoffperoxid ($H_2O_2$) von außen zugeführt werden muß, um Oxidationsprozesse durch UV-Licht zu erreichen, da dies innerhalb der Vorrichtung zeitgleich zur angestrebten Reaktion Wasserstoffperoxid erzeugt. Die vorliegende Erfindung ermöglicht, reine Flüssigkeiten in einem von der jeweiligen Anwendungsform abhängigen Zeitlimit ohne weitere Chemikalienzusätze oder kostenintensive Hilfsmittel herzustellen. Dadurch resultieren Vorrichtungen auf deutlich höheren Qualitätsniveau und mit einem deutlich höheren Wirkungsgrad.

**Patentansprüche**

1. Vorrichtung (100) zur Aufbereitung von Flüssigkeiten zwecks Sterilisation und Elimination von darin enthaltenen organischen Stoffen mittels UV-Licht, umfassend

- ein ein- oder mehrteiliges Gehäuse (110) mit einem Flüssigkeitszulauf (9) und einem Flüssigkeitsablauf (11) für einen Durchlauf von aufzubereitender Flüssigkeit durch das Gehäuse (110),
- mindestens eine UV-Lichtquelle (1) in dem Gehäuse (110),
- mindestens eine Ultraschallquelle (8; 12) in dem Gehäuse (110),
- eine Entgasungseinrichtung (15) zur Entgasung von Flüssigkeit,

**dadurch gekennzeichnet, daß** das Gehäuse (110) die Gestalt eines Zylinders aufweist und in Längsrichtung des Zylinders in zwei Kammern (7, 7') unterteilt ist, sich die UV-Lichtquelle (1) in Längsrichtung des Zylinders durch beide Kammern (7, 7') erstreckt, ein erstes für LTV-Licht durchlässiges Schutzrohr (2) die UV-Lichtquelle (1) durch beide Kammern (7, 7') hindurch umgibt, ein zweites Rohr (4) mit einem größeren Innendurchmesser als der Außehdurchmesser des ersten Schutzrohres (2) das erste Schutzrohr (2) unter Bildung eines Ringspalts durch beide Kammern (7, 7') hindurch umgibt, wobei an beiden Enden des zweiten Rohres jeweils mindestens eine Öffnung (10, 10') zur Herstellung einer Flüssigkeitsverbindung zwischen dem Ringspalt (5) und der Außenseite des zweiten Rohres vorgesehen ist und der Flüssigkeitszulauf (9) fern von der mindestens einen Öffnung (10, 10') an einem Ende des zweiten Rohres in einer der beiden Kammern (7, 7') vorgesehen ist und der Flüssigkeitszulauf (9) fern von der mindestens einen Öffnung (10, 10') an dem anderen Ende des zweiten Rohres in der anderen Kammer (7, 7') vorgesehen ist.

2. Vorrichtung (100) nach Anspruch 1, **dadurch gekennzeichnet, daß** die Entgasungseinrichtung (15) eine mechanische Entgasungseinrichtung umfaßt.

3. Vorrichtung (100) nach Anspruch 1, **dadurch gekennzeichnet, daß** die Entgasungseinrichtung (15) einen mechanischen Luftabscheider umfaßt.

4. Vorrichtung (100) nach - Anspruch 1, **dadurch gekennzeichnet, daß** die Entgasungseinrichtung (15) eine Ultraschallquelle, einen Membrankontaktor, eine Vakuum-Entgasungseinrichtung oder eine Düse in dem Flüssigkeitszulauf (9) umfaßt.

5. Vorrichtung nach einem der Ansprüche 1 bis 4, **dadurch gekennzeichnet, daß** die Entgasungseinrichtung (15) innerhalb des Gehäuses (110), vorzugsweise im Bereich des Flüssigkeitszulaufs (9), vorgesehen ist.

6. Vorrichtung (100) nach einem der Ansprüche 1 bis 5, **dadurch gekennzeichnet, daß** sich in der Kammer (7, 7') mit dem Flüssigkeitszulauf (9) mindestens ein Kavitation erzeugendes Sieb (16) oder Filter befindet.

**7.** Vorrichtung (100) nach Anspruch 6, **dadurch gekennzeichnet, daß** sich das mindestens eine Sieb (16) in der Kammer (7, 7') im Bereich des Flüssigkeitszulaufes (9) befindet.

**8.** Vorrichtung (100) nach einem der vorangehenden Ansprüche, **dadurch gekennzeichnet, daß** die mindestens eine Ultraschallquelle (8; 12) zur Emission von Ultraschall mit einer Frequenz > 18 kHz und/oder einer Leistung > 1 Watt/cm$^2$ im Ringspalt gestaltet ist.

**9.** Vorrichtung (100) nach einem der vorangehenden Ansprüche, **dadurch gekennzeichnet, dass** die mindestens eine Ultraschallquelle (8) oder mindestens eine Ultraschallquelle (8) so angeordnet ist, dass sie im Betrieb im wesentlichen senkrecht zur Längsrichtung des Zylinders Ultraschall vom Gehäuse (110) in Richtung auf die UV-Lichtquelle (1) einstrahlt.

**10.** Vorrichtung (100) nach einem der vorangehenden Ansprüche, **dadurch gekennzeichnet, daß** mindestens eine Ultraschallquelle (12) so angeordnet ist, daß sie im Betrieb im wesentlichen parallel zur Längsrichtung des Zylinders Ultraschall im Bereich zwischen dem zweiten Rohr und dem Gehäuse (110) einstrahlt.

**11.** Vorrichtung (100) nach einem der vorangehenden Ansprüche, **dadurch gekennzeichnet, daß** jeder der beiden Kammern (7, 7') jeweils mindestens eine Ultraschallquelle (8; 12) zum Bestrahlen der aufzubereitenden Flüssigkeit mit Ultraschall zugeordnet ist.

**Claims**

**1.** An apparatus (100) for processing liquids for the purposes of sterilization and the elimination of organic substances contained therein by means of UV light, comprising:

> ● a one- or multi-part housing (110) with a liquid inlet (9) and a liquid outlet (11) for the passage of liquid to be prepared through the housing (110);
> ● at least one UV light source (1) in said housing (110);
> ● at least one ultrasound source (8; 12) in said housing (110);
> ● a degassing device (15) for degassing liquid;

**characterized in that** the housing (110) is cylindrical in shape and is divided in the longitudinal direction of the cylinder into two chambers (7, 7'), **in that** the UV light source (1) extends in the longitudinal direction of the cylinder through both chambers (7, 7'), **in that** a first UV-transparent protective tube (2) passing through both chambers (7, 7') surrounds the UV light source (1), **in that** a second tube (4) with a larger internal diameter than the external diameter of the first protective tube (2) surrounds the first protective tube (2) forming an annular gap through both chambers (7, 7'), wherein at least one opening (10, 10') is provided at both ends of the second tube to provide liquid communication between the annular gap (5) and the outside of the second tube, and **in that** the liquid inlet (9) is located at a substantial distance from the at least one opening (10, 10') at one end of the second tube in one of the two chambers (7, 7'), and **in that** the liquid inlet (9) is located at a substantial distance from the at least one opening (10, 10') at the other end of the second tube in the other chamber (7, 7').

**2.** An apparatus (100) according to claim 1, **characterized in that** the degassing device (15) comprises a mechanical degassing device.

**3.** An apparatus (100) according to claim 1, **characterized in that** the degassing device (15) comprises a mechanical air separator.

**4.** An apparatus (100) according to claim 1, **characterized in that** the degassing device (15) comprises an ultrasound source, a membrane contactor, a vacuum degassing device or a nozzle in the liquid inlet (9).

**5.** An apparatus according to one of claims 1 to 4, **characterized in that** the degassing device (15) is provided inside the housing (110), preferably in the region of the liquid inlet (9).

**6.** An apparatus (100) according to one of claims 1 to 5, **characterized in that** at least one sieve (16) or filter which produces cavitation is present in the chamber (7, 7') with the liquid inlet (9).

**7.** An apparatus (100) according to claim 6, **characterized in that** the at least one sieve (16) in the chamber (7, 7') is in the region of the liquid inlet (9).

**8.** An apparatus (100) according to one of the preceding claims, **characterized in that** the at least one ultrasound source (8; 12) is configured to emit ultrasound at a frequency of > 18 kHz and/or a power of > 1 Watt/cm$^2$ in the annular gap.

**9.** An apparatus (100) according to one of the preceding claims, **characterized in that** the at least one ultrasound source (8) or at least one ultrasound source (8) is arranged such that in operation it directs ultrasound from the housing (110) in the direction of the UV light source (1) essentially perpendicular to the longitudinal direction of the cylinder.

10. An apparatus (100) according to one of the preceding claims, **characterized in that** at least one ultrasound source (12) is arranged such that in operation it directs ultrasound essentially parallel to the longitudinal direction of the cylinder in the region between the second tube and the housing (110).

11. An apparatus (100) according to one of the preceding claims, **characterized in that** each of the two chambers (7, 7') has assigned thereto at least one ultrasound source (8; 12) to irradiate the liquid to be prepared with ultrasound.

**Revendications**

1. Dispositif (100) pour le traitement de liquides à des fins de stérilisation et d'élimination de substances organiques y étant contenues en utilisant la lumière V, comprenant :

   - un boîtier (110) en une ou plusieurs parties, avec une amenée de liquide (9) et une évacuation de liquide (11), pour un passage de liquide à réparer à travers le boîtier (110),
   - au moins une source de lumière UV (1) dans le boîtier (110),
   - au moins une source d'ultrasons (8 ; 12) dans le boîtier (110),
   - un dispositif de dégazage (15), pour le dégazage de liquide,

   **caractérisé en ce que** le boîtier (110) présente la forme d'un cylindre et est subdivisé en direction longitudinale du cylindre, en deux chambres (7, 7'), que la source de lumière UV (1) s'étend en direction longitudinale du cylindre, à travers les deux chambres (7, 7'), un premier tube de protection (2), perméable à la lumière UV, entoure la source de lumière W (1) en passant à travers les deux chambres (7, 7'), un deuxième tube (4), d'un diamètre intérieur plus grand que le diamètre extérieur du premier tube de protection (2), entoure le premier tube de protection (2) en formant un intervalle annulaire à travers les deux chambres (7, 7'), à chacune des deux extrémités du deuxième tube étant prévue au moins une ouverture (10, 10'), pour établir une liaison de liquide entre l'intervalle annulaire (5) et la face extérieure du deuxième tube, et l'amenée de liquide (9) étant prévue loin de la au moins une ouverture (10, 10'), à une extrémité du deuxième tube, dans l'une des deux chambres (7, 7'), et l'amenée de liquide (9) étant prévue loin de la au moins une ouverture (10, 10'), à l'autre extrémité du deuxième tube, dans l'autre chambre (7, 7').

2. Dispositif selon la revendication 1, **caractérisé en ce que** le dispositif de dégazage (15) comprend un dispositif de dégazage mécanique.

3. Dispositif selon la revendication 1, **caractérisé en ce que** le dispositif de dégazage (15) comprend un séparateur d'air mécanique.

4. Dispositif selon la revendication 1, **caractérisé en ce que** le dispositif de dégazage (15) comprend une source d'ultrasons, un contacteur à membrane, un dispositif de dégazage sous vide ou une buse placée dans l'amenée de liquide (9).

5. Dispositif selon l'une des revendications 1 à 4, **caractérisé en ce que** le dispositif de dégazage (15) est prévu à l'intérieur du boîtier (110), de préférence dans la zone de l'amenée de liquide (9).

6. Dispositif selon l'une des revendications 1 à 5, **caractérisé en ce qu'**au moins un tamis (16) ou un filtre, générant de la cavitation, se trouve dans la chambre (7, 7') avec l'amenée de liquide (9).

7. Dispositif selon la revendication 6, **caractérisé en ce que** le au moins un tamis (16) placé dans la chambre (7, 7') se trouve dans la zone de l'amenée de liquide (9).

8. Dispositif (100) selon l'une des revendications précédentes, **caractérisé en ce que** la au moins une source d'ultrasons (8, 12) est configurée pour l'émission d'ultrasons d'une fréquence > 18 kHz et/ou d'une puissance > 1 Watt/cm$^3$ dans l'intervalle annulaire.

9. Dispositif (100) selon l'une des revendications précédentes, **caractérisé en ce que** la au moins une source d'ultrasons (8) ou au moins une source d'ultrasons (8) est disposée de manière qu'elle introduise en fonctionnement, sensiblement perpendiculairement à la direction longitudinale du cylindre, des ultrasons depuis le boîtier (110), en direction de la source de lumière UV (1).

10. Dispositif (100) selon l'une des revendications précédentes, **caractérisé en ce que** la au moins une source d'ultrasons (12) est disposée de manière qu'elle introduise en fonctionnement, sensiblement parallèlement à la direction longitudinale du cylindre, des ultrasons dans la zone située entre le deuxième tube et le boîtier (110).

11. Dispositif (100) selon l'une des revendications pré-

13       **EP 1 862 185 B1**       14

cédentes, **caractérisé en ce qu'**à chacune des deux chambres (7, 7') est associée au moins une source d'ultrasons (8 ; 12), pour irradier en ultrasons le liquide à préparer.

**8**

Fig. 1

EP 1 862 185 B1

Fig. 2

**Fig. 3**

EP 1 862 185 B1

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202005009923 U1